**Europäisches Patentamt**

**European Patent Office**

**Office européen des brevets**

(19)

(11) Veröffentlichungsnummer : **0 373 097 B1**

(12) ## EUROPÄISCHE PATENTSCHRIFT

(45) Veröffentlichungstag der Patentschrift :
**05.08.92 Patentblatt 92/32**

(51) Int. Cl.⁵ : **A61F 2/24**, A61L 27/00

(21) Anmeldenummer : **89730210.5**

(22) Anmeldetag : **26.10.89**

(54) **Herzklappenprothese.**

(30) Priorität : **29.11.88 DE 8815082 U**

(43) Veröffentlichungstag der Anmeldung :
**13.06.90 Patentblatt 90/24**

(45) Bekanntmachung des Hinweises auf die
Patenterteilung :
**05.08.92 Patentblatt 92/32**

(84) Benannte Vertragsstaaten :
**AT CH DE FR GB IT LI**

(56) Entgegenhaltungen :
**DD-A- 133 518**
**US-A- 4 240 161**
**US-A- 4 326 304**

(73) Patentinhaber : **BIOTRONIK Mess- und**
**Therapiegeräte GmbH & Co Ingenieurbüro**
**Berlin**
**Woermannkehre 1**
**W-1000 Berlin 47 (DE)**

(72) Erfinder : **Bolz, Armin Dipl.-Phys.**
**Mozartstrasse 40**
**W-8520 Erlangen (DE)**
Erfinder : **Schaldach, Max Prof. Dr.-Ing.**
**Turnstrasse 5**
**W-8520 Erlangen (DE)**

(74) Vertreter : **Christiansen, Henning, Dipl.-Ing.**
**Patentanwalt CHRISTIANSEN Pacelliallee**
**43/45**
**W-1000 Berlin 33 (DE)**

EP 0 373 097 B1

## Beschreibung

Die Erfindung betrifft eine Herzklappenprothese der im Oberbegriff des Anspruchs 1 angegebenen Art. Eine solche Prothese ist aus der Druckschrift US-A-4 326 304 bekannt.

Bei derartigen künstlichen Herzklappen besteht das Problem, daß der die Ventilklappe tragende Ringkörper möglichst unverformt bleiben soll, wenn die Ventilklappe eingesetzt wird, damit er einerseits keine dauerhafte Verformung erleidet und andererseits Oberflächenbeschichtungen nicht beschädigt werden.

Der Erfindung liegt die Aufgabe zugrunde, bei einer Herzklappenprothese der eingangs genannten Gattung Maßnahmen anzugeben, welche es ermöglichen, die Ventilklappe ohne Verformung des Ringkörpers zu montieren.

Diese Aufgabe wird mit den kennzeichnenden Merkmalen des Anspruchs 1 gelöst.

Die Erfindung beruht auf der Erkenntnis, daß, wenn der Ringkörper mit einer Nut in der Weise versehen ist, die Ventilklappe in das Lager ausschließlich in einer Stellung eingesetzt werden kann, welche sie hydrodynamisch im inplantierten Zustand nicht einnehmen kann, und so einerseits eine Verformung des äußeren Ringkörpers vermieden wird und andererseits auch sichergestellt ist, daß die Ventilklappe im Betriebszustand nicht aus ihrer Lagerung gelangen kann.

Dies ist gewährleistet wenn bei der Herzklappenprothese erfindungsgemäß der Ringkörper an seiner Innenwandung an die erste Ausnehmungen jeweils anschließende weitere Ausnehmungen aufweist, welche Zum Einsetzen der Ventilklappe in den Ring-Körper einen Durchlaß für die konvexen Ansätze zum oberen und/oder unteren Rand des Ringes zur in einer Solchen Position der Ventilklappe bilden, die sich jenseits einer Position befindet, welche die Klappe hydrodynamisch einnehmen kann, die Sicherheit des Patienten in jeder Weise gewährleistet ist, ohne daß die Montage aufwendiger wäre.

Bei einer vorteilhaften Weiterbildung der Erfindung ist der Durchlaß zur in einer Winkelstellung der Ventilklappe für die konvexen Ansätze passierbar, die sich, ausgehend von der Betriebsstellung, jenseits der durch einen ersten bzw. zweiten Anschlag begrenzten offenen bzw. geschlossenen Position befindet. Hierdurch ist gewährleistet, daß die Winkelposition zum Einsetzen im implantierten Zustand nicht wieder erreicht wird. Der erste Anschlag für die Ventilklappe in ihrer geöffneten Position befindet sich dabei bevorzugt in Strömungsrichtung hinter dem Lager der Ventilklappe und der zweite Klappenanschlag für die geschlossenen Position wird durch eine an der Innenwandung des Ringkörpers vorgesehene Kante gebildet. Beide Anschläge weisen dabei bevorzugt im Querschnitt die Form eines Widerhakens auf. In günstiger Weiterbildung weist der Ringkörper in tangentialer Richtung entlang der Innenwandung in einem Bereich zwischen den beiden Lagern eine die Klappenbewegung in Richtung über die geschlossene Position hinaus begrenzende Stufe auf.

Besonders vorteilhaft ist außerdem die Verwendung eines halbleitenden Materials als Beschichtung des Implantats, weil damit Elektronenströme verhindert werden, welche eine Fibrinaktivierung begünstigen. Dazu muß nur eine äußerst dünne Oberflächenschicht aus Halbleitermaterial bestehen. Die entsprechenden Beschichtungen lassen sich kostengünstig durch PVD- oder CVD-Verfahren erzeugen. Dabei ist insbesondere die Erzeugung von amorphem SiC nach dem sogenannten Plasma-CVD-Verfahren (Plasma assisted chemical vapor deposition) günstig. Auf diese Weise lassen sich Schichten sehr geringer elektrischer Zustandsdichte erzeugen.

Insbesondere sind noch folgende vorteilhafte Weiterbildungen günstig:

Die porenfreie Halbleiterbeschichtung besteht entweder aus einem amorphen oder einem mikrokristallinen Werkstoff. Die Rauhtiefe der Oberfläche beträgt weniger als 0,1 $\mu$m. Dadurch wird die Anlagerung bzw. Zerstörung korpuskulärer Bestandteile des Blutes und die damit verbundene Aktivierung des Gerinnungssystemes verhindert.

Die Lösung basiert auf der Erkenntnis, daß auf dem energetischen Niveau der elektronischen Proteinzustände die Zustandsdichte der Bechichtung gering ist. Damit werden thrombogene Ladungsaustauschprozesse zwischen gerinnungsspezifischen Proteinen und der Implantatoberfläche verhindert.

Bevorzugt wird zur antithrombogenen Beschichtung amorphes Siliziumkarbid (a-SiC:H), ein Abscheidungsprodukt eines Gemisches enthaltend Silan ($SiH_4$) und/oder Methan ($CH_4$), verwendet. Entsprechend kann auc Siliziumoxis (a-SiO:H) vorteilhaft verwendet werden.

Die Beschichtung ist insbesondere möglichst porenfrei ausgebildet. Dadurch erhöht sich auch deren Stabilität. Statt Siliziumkarbid kann auch Siliziumnitrid (a-SiN:H) benutzt werden, das insoweit übereinstimmende Oberflächeneigenschaften aufweist.

Vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:

Figur 1 den Ringkörper der erfindungsgemäßen Herzklappenprothese in der Draufsicht,

Figur 2 eine Schnittdarstellung des Ausführungsbeispiels gemäß Figur 1,

Figuren 3a und b einen weiteren Schnitt durch das in Figur 1 dargestellte Ausführungsbeispiel mit zwei möglichen Stellungen des erfindungsgemäßen Ventilkörpers sowie

Figur 4 eine vergrößerte Schnittdarstellung.

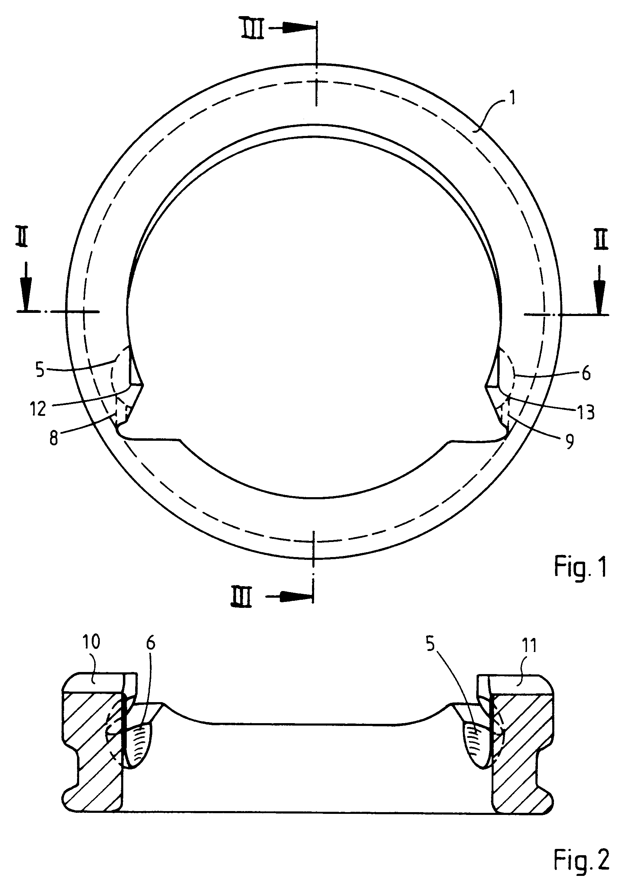

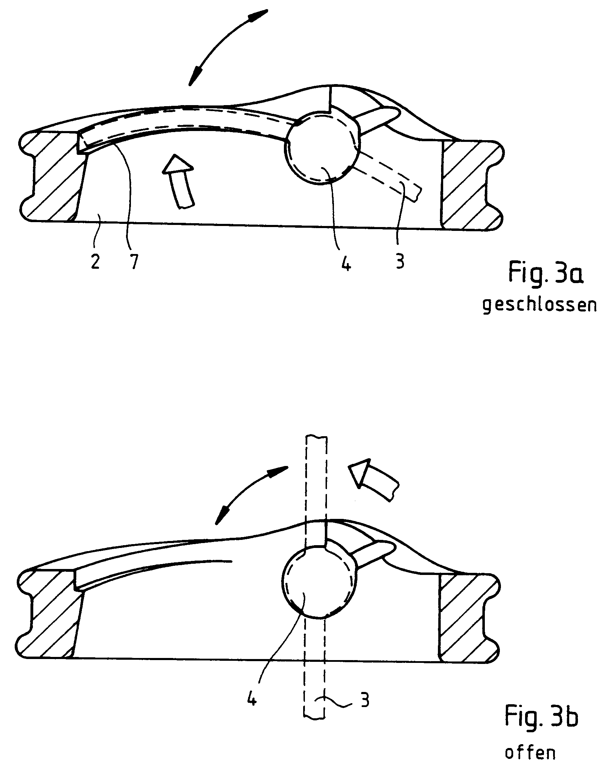

Bei dem in Figur 1 in der Draufsicht dargestellten Ausführungsbeispiel der erfindungsgemäßen Herzklappenprothese ist lediglich der Ringkörper 1 wiedergegeben. Die Ventilklappe selbst ist aus Gründen der Übersichtlichkeit fortgelassen. Die Beschreibung soll unter gleichzeitiger Bezugnahme auf die Figuren 2 und 3 erfolgen, welche die in Figur 1 angedeuteten Schnittansichten (II bzw. III) wiedergeben. Der Ringkörper 1 weist eine umlaufende Einrillung 2 zur Befestigung der Herzklappenprothese mittels Fissur auf. Die in den Figuren 3a und 3b in der offenen (Figur 3b) und der geschlossenen (Figur 3a) Position dargestellte Ventilklappe 3 ist mittels balligen Ansätzen 4, die Stummel für die Drehlagerung bilden, beweglich in entsprechende Ausnehmungen 5 und 6 des Ringkörpers 1 eingeschnappt. Die balligen Ansätze 4 - von denen in der Ansicht gemäß Figuren 3a und b lediglich der vordere sichtbar ist, bilden gemeinsam die Enden einer Drehachse für die Ventilklappe 3, welche einer Sekante der das Innere des Ringkörpers 1 ausfüllenden Kreisscheibe entspricht. Auf diese Weise überwiegen die auf den größeren von der Sekante abgeteilten Teil der Kreisscheibe wirkenden Kräfte, so daß die Ventilklappe 4 mit dem Blutstrom geöffnet oder geschlossen wird. In der geschlossenen Stellung lehnt sich die Ventilklappe 4 an eine Kante 7 an, welche sich an der Innenwandung des Ringkörpers 1 parallel zum Rand erstreckt. Die Anschlagkante 7 dehnt sich über einen Bereich zwischen den Ausnehmungen 5 und 6 aus, der bevorzugt an dem Bereich des Ringkörpers gelegen ist, der von der zwischen den Ansätzen 4 gelegenen Drehachse den größeren Abstand aufweist.

Die Kante 7 ist im Querschnitt widerhakenförmig, d.h. der Innendurchmesser des Ringkörpers erweitert sich - in Schließrichtung der Ventilklappe gesehen - jenseits der Anschlagkante 7 für die geschlossene Stellung allmählich wieder. Auf diese Weise läßt sich die entsprechend elastische Ventilklappe 3 zum Einsetzen in den Ringkörper auch von einer Position, die jenseits der geschlossenen Position gelegen ist, über die mit der Anschlagkante 7 verbundene Stufe in die geschlossene Position drücken, aus der sie im implantierten Zustand durch die hydrodynamischen Kräfte nicht hinausgelangen kann.

Damit die Ansätze 4 der Herzklappe ohne wesentliche Verformung des Ringkörpers in die konkaven Ausnehmungen 5 und 6 gelangen können, sind an diese anschließende Ausnehmungen 8 bzw. 9 vorgesehen, welche einen Durchlaß für die noppenförmigen, balligen axialen Erhebungen 4 zum Rand des Ringkörpers hin bilden. Diese ergänzenden Ausnehmungen bzw. Aussparungen 8 und 9 können als durch die Spur eines Fräsers mit den Abmessungen der balligen Erhebungen 4 entstanden gedacht werden. Wäre der Ringkörper 1 aus plastischem Material und die Ventilklappe einschließlich Ansätzen 4 starr, so würden sich diese in der entsprechenden Weise in das Material einformen.

Die Ausnehmungen bzw. Aussparungen 8 bzw. 9 enden bevorzugt im Bereich von Anformungen 10 und 11 an den Ringkörper 1, welche im Bereich der Ausnehmungen 5,6 die axiale Länge des Ringkörpers 1 vergrößern, so daß die konkaven, kuppelförmigen Ausnehmungen 5 und 6 Platz finden. Im Bereich dieser Anformungen sind weiterhin Anschläge 12 und 13 vorgesehen, welche Aussparungen begrenzen, in die der Rand des scheibenförmigen Teils der Ventilklappe 3 im geöffneten Zustand (Figur 3b) gelangt, so daß die Ventilklappe nicht über die vertikale Position hinausgelangen kann. Auch diese Anschläge 12 und 13 mit den entsprechenden Aussparungen weisen eine Form auf, die so entstanden gedacht sein kann, daß in dem plastischen Ringkörper 1 die Ventilklappe aus der geschlossenen Stellung um 90° in die geöffnete Stellung gedreht wird, wobei der von der Drehachse entfernt gelegene größte Durchmesser das Material des Ringkörpers 1 verdrängt.

Die die Ausnehmung bzw. Aussparung 8 bzw. 9 begrenzende Kante, welche jenseits des Anschlags 12 bzw. 13 in der Draufsicht gemäß Figur 1 erkennbar ist, weist eine nach außen gerichtete Neigung auf, so daß in der Draufsicht der Anschlag 12 mit den daran anschließenden Kanten insgesamt die Form eines Widerhakens erhält. Es ist erkennbar, daß beim Einschieben der Ventilklappe in der geöffneten Stellung die Kanten über die an die Anschlagfläche 12 bzw. 13 nach außen hin anschließende Kante hinweggleiten und im Bereich der Anschlagfläche 11 rasten. Auf diese Weise ist die Ventilklappe ohne wesentliche Verformung des Ringkörpers in diesen einfügbar und kann durch die Wirkung der Anschlagflächen 12 und 13 in der geöffneten Stellung nicht wieder in die Position gelangen, in der sie eingefügt wurde. Das Einsetzen kann auch ohne Verformung des Ringkörpers erfolgen, wobei die Ventilklappe zum Überwinden der Rastwirkung der Anschlagflächen 11 und 12 lediglich geringfügig durchgebogen werden muß, womit, da die Verformung gleichmäßig über den gesamten Durchmesser der Herzklappe erfolgen kann, keine größeren lokalen Materialspannungen verbunden sind.

In den Figuren 3a und 3b ist der Bereich, in dem die Ventilklappe im Betrieb frei schwingen kann, durch die Pfeile mit einfachen Linien und die zum Einschwenken beim Einsetzen erforderliche Bewegung mit doppelt ausgezogenen Pfeilen dargestellt.

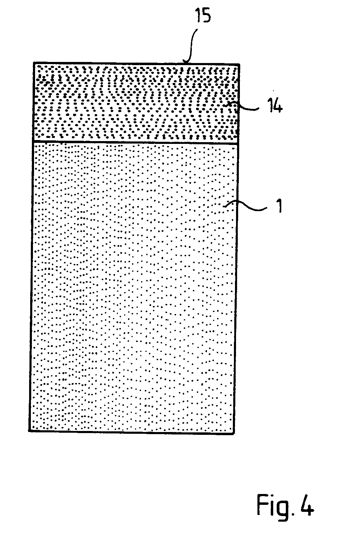

Bei dem in Figur 4 dargestellten Ausführungsbeispiel eines erfindungsgemäßen kardiovaskulären Implantats ist dessen Schichtaufbau im Querschnitt wiedergegeben. Auf der Oberfläche eines Implantats, gebildet durch ein aus einem Grundwerkstoff bestehendes Teil 1, befindet sich eine porenfreie Beschichtung 14 aus amorphem Siliziumkarbid (a-SiC:H) als Halbleiterwerkstoff, das ein Abscheidungsprodukt eines Gemisches enthaltend Silan ($SiH_4$) und/oder Methan ($CH_4$) bildet und dessen Oberfläche 14 antithrombogen ausgebildet ist. Bei dem Teil 1 handelt es sich beispielsweise um den äußeren Ring einer Herzklappenprothese oder die nicht isolierten (aktiven) Bereiche einer Herzschrittmacherelektrode.

Die Beschichtung 14 ist maximal 1 μm dick und hat einen vergleichsweise hohen E-Modul und einen relativ geringeren elastischen Bereich. Die Oberfläche 14 weist eine Rauhtiefe von ca. 0,1 μm oder weniger auf. Die Zustandsdichte der Beschichtung 13 liegt im Bereich des energetischen Niveaus der Proteinzustände im Blut, also bei $10^{18}$ $cm^{-3}eV^{-1}$, oder niedriger. Die Ausbildung der Oberfläche 15 verhindert direkte Ladungsaustauschprozesse zwischen gerinnungsspezifischen Proteinen und der Implantatoberfläche.

Bei einer weiteren Ausführungsvariante befindet sich auf einem aus einem Grundwerkstoff bestehenden Teil 1 eine Schicht 14 aus einem mikrokristallinen Halbleiterwerkstoff, insbesondere Siliziumnitrid (a-SiN:H). Im übrigen weist die Oberfläche 15 dieses Ausführungsbeispiels entsprechende Eigenschaften wie das zuvor beschriebene auf.

Die Erfindung beschränkt sich in ihrer Ausführung nicht auf das vorstehend angegebene bevorzugte Ausführungsbeispiel, Soudern kann auch andere Varianten enthalten welche unter die folgenden Ansprüche fallen.

## Patentansprüche

1. Herzklappenprothese mit
   einer drehbar gelagerten scheibenförmigen Ventilklappe (3), welche zwei in den Schnittpunkten einer Sekante mit dem Scheibenrand einander gegenüberliegende, nach außen gerichtete erhabene, insbesondere kugelkappenförmig konvexe Ansätze (4) aufweist,
   und
   einem Ringkörper (1), der an seiner Innenwandung an die konvexen Ansätze angepaßte halbschalenförmige erste Ausnehmungen (5,6) aufweist, welche die Lagerung für die Ventilklappe bilden,
   **dadurch gekennzeichnet,**
   daß der Ringkörper (1) an seiner Innenwandung an die ersten Ausnehmungen (5, 6) jeweils anschließende weitere Ausnehmungen (8, 9) aufweist, welche Zum Einsetzender Ventilklappe (3) in den Ringkörper (1) einen Durchlaß für die konvexen Ansätze (4) zum Rand des Ringes nur in einer solchen Position der Ventilklappe (3) bilden, die sich jenseits der voll geöffneten oder voll geschlossenen Position befindet.

2. Herzklappenprothese nach Anspruch 1, **dadurch gekennzeichnet,** daß die den Durchlaß bildende weitere Ausnehmungen (8, 9) nur in einer Winkelstellung der Ventilklappe für die konvexen Ansätze (4) passierbar sind, die sich ausgehend von der Betriebsstellung, jenseits der durch einen ersten bzw. zweiten Anschlag (12,13 bzw. 7) begrenzten offenen (Fig. 3b) bzw. geschlossenen (Fig. 3a) Position befindet.

3. Herzklappenprothese nach einem der vorangehenden Ansprüche, **dadurch gekennzeichnet,** daß der erste Anschlag (12, 13) in der geöffneten Position der Ventilklappe sich in Strömungsrichtung hinter dem Lager der Ventilklappe (3) befindet.

4. Herzklappenprothese nach einem der vorangehenden Ansprüche, **dadurch gekennzeichnet,** daß der zweite Anschlag durch eine an der Innenwandung des Ringkörpers vorgesehene Kante (7) gebildet wird.

5. Herzklappenprothese nach einem der Ansprüche 2 bis 4, **dadurch gekennzeichnet,** daß der erste und/oder zweite Anschlag (7, 12 bzw. 13) im Querschnitt Form eines Widerhakens aufweist.

6. Herzklappenprothese nach einem der vorangehenden Ansprüche, **dadurch gekennzeichnet,** daß die weiteren Ausnehmungen (8, 9) derart groß bemessen sind, daß ein Einsetzen der Ventilklappe (3) ohne wesentliche Verformung des Ringkörpers (1) erfolgen kann.

7. Herzklappenprothese nach einem der vorangehenden Ansprüche, **dadurch gekennzeichnet,** daß der zweite Anschlag (7) in Form einer innerhalb des Ringkörpers (1) in tangentialer Richtung entlang der Innenwandung in einem Bereich zwischen den beiden Lagern verlaufenden, eine die Klappenbewegung in Richtung über die geschlossene Position hinaus begrenzende Stufe ausgebildet ist.

8. Herzklappenprothese nach einem der vorangehenden Ansprüche, **dadurch gekennzeichnet,** daß mindestens der Ringkörper (1) eine Beschichtung (14) aus bestehend aus einem halbleitenden Material, vorgesehen ist, wobei das halbleitende Material amorph, insbesondere mikrokristallin ist.

9. Herzklappenprothese nach Anspruch 8, **dadurch gekennzeichnet,** daß die Beschichtung (14) eine Dikke im Bereich von 1 μm, maximal 10 μ, bei einer Rauhigkeit im Bereich von insbesondere 0,1 μ aufweist.

10. Herzklappenprothese nach einem der Ansprüche 8 oder 9, **dadurch gekennzeichnet,** daß die Zustandsdichte der Beschichtung (14) auf dem energetischen Niveau der Proteinzustände gering ist, so daß direkte Ladungsaustauschprozesse zwischen gerinnungsspezifischen Proteinen und der Implantatoberfläche (15) verhindert werden.

11. Herzklappenprothese nach Anspruch 8, **dadurch gekennzeichnet,**
daß die Beschichtung (13) aus pyrolytischem oder amorphem Siliziumkarbid (a-SiC:H) besteht, wobei das amorphe Siliziumkarbid insbesondere ein Abscheidungsprodukt eines Gemisches, enthaltend Silan (SiH4) und/oder Methan (CH4), bildet, oder
daß die Beschichtung (13) aus amorphem Siliziumnitrid (a-SiN:H) oder Siliziumoxid (a-SiO:H) besteht.


**Claims**

1. Heart valve prosthesis having
    a rotatably supported, disk-shaped valve flap (3) with two outwardly directed, raised, in particular spherical cap-like convex projections (4), which lie opposite one another in the points of intersection of a secant with the disk rim,
and
    a ring element (1), which has on its inner wall half shell-shaped first recesses (5, 6), which are adapted to the convex projections and form the bearing for the heart flap,
**characterised in that**
the ring element (1) has on its inner wall further recesses (8, 9) which in each case adjoin the first recesses (5, 6) and, for insertion of the valve flap (3) into the ring element (1), form a passage for the convex projections (4) towards the rim of the ring only when the valve flap (3) is in a position lying beyond the fully open or fully closed position.

2. Heart valve prosthesis according to claim 1, **characterised in that** the further recesses (8, 9) forming the passage are traversable by the convex projections (4) only when the valve flap is in an angular position which, originating from the operating position, lies beyond the open position (Fig.3b) or the closed position (Fig.3a) delimited by a first or second stop (12, 13 or 7).

3. Heart valve prosthesis according to one of the preceding claims, **characterised in that**, in the open position of the valve flap, the first stop (12, 13) lies downstream of the bearing of the valve flap (3).

4. Heart valve prosthesis according to one of the preceding claims, characterised in that the second stop is formed by an edge (7) provided on the inner wall of the ring element.

5. Heart valve prosthesis according to one of claims 2 to 4, **characterised in that** the first and/or second stop (7, 12 and/or 13) is barb-shaped in cross-section.

6. Heart valve prosthesis according to one of the preceding claims, **characterised in that** the further recesses (8, 9) are of sufficiently large dimensions to allow the valve flap (3) to be inserted without substantial deformation of the ring element (1).

7. Heart valve prosthesis according to one of the preceding claims, **characterised in that** the second stop (7) takes the form of a shoulder which extends inside the ring element (1) in a tangential direction along the inner wall in a region between the two bearings and delimits the flap movement in the direction beyond the closed position.

8. Heart valve prosthesis according to one of the preceding claims, **characterised in that** at least the ring element (1) is provided with a coating (14) made of a semiconductive material, said semiconductive material being amorphous, in particular microcrystalline.

9. Heart valve prosthesis according to claim 8, **characterised in that** the coating (14) has a thickness in the region of 1 $\mu$m, at most 10 $\mu$m, given a surface roughness in the region of, in particular, 0.1 $\mu$.

10. Heart valve prosthesis according to one of claims 8 or 9, **characterised in that** the state density of the coating (14) at the energy level of the protein states is low so that direct charge exchange processes between coagulation-specific proteins and the implant surface (15) are avoided.

11. Heart valve prosthesis according to claim 8, **characterised in that**
the coating (13) is made of pyrolytic or amorphous silicon carbide (a-SiC:H), with the amorphous silicon carbide in particular forming a precipitation product of a mixture containing monosilane (SiH4) and/or methane (CH4), or
the coating (13) is made of amorphous silicon nitride (a-SiN:H) or silicon oxide (a-SiO:H).


**Revendications**

1. Prothèse de valvule cardiaque comprenant :
    un clapet (3) de valvule en forme de disque, monté rotatif, qui présente deux tétons (4) convexes, notamment en forme de calotte de sphère, qui sont situés l'un à l'opposé de l'autre aux points d'intersection d'une sécante et du bord du disque, et en saillie vers l'extérieur, et

un corps annulaire (1) qui présente, sur sa paroi interne, des premiers évidements hémisphériques (5, 6) adaptés aux tétons convexes, et qui forment la portée de rotation du clapet de valvule, **caractérisée en ce que**

le corps annulaire (1) présente sur sa paroi interne des évidements additionnels (8, 9) qui se raccordent respectivement aux premiers évidements (5, 6) et qui, pour la mise en place du clapet (3) de valvule dans le corps annulaire (1), forment pour les tétons convexes (4) un passage qui mène au bord de l'anneau, uniquement dans une position du clapet (3) de valvule qui se trouve au-delà de la position entièrement ouverte ou de la position entièrement fermée.

2. Prothèse de valvule cardiaque selon la revendication 1, **caractérisée en ce que** les évidements additionnels (8, 9) qui forment le passage ne peuvent être franchis par les tétons convexes (4) que dans une position angulaire du clapet de valvule qui, lorsqu'on part de la position de service, se trouve au-delà de la position ouverte (figure 3b) limitée par une première butée (12, 13) ou au-delà d'une position fermée (figure 3a), par une deuxième butée (7).

3. Prothèse de valvule cardiaque selon une des revendications précédentes, **caractérisée en ce que** la première butée (12, 13), dans la position ouverte du clapet, se trouve en aval de la portée de rotation du clapet (3) de valvule dans le sens de l'écoulement.

4. Prothèse de valvule cardiaque selon une des revendications précédentes, **caractérisée en ce que** la deuxième butée est formée par une arête (7) prévue le long de la paroi interne du corps annulaire.

5. Prothèse de valvule cardiaque selon une des revendications 2 à 4, **caractérisée en ce que** la première et/ou la deuxième butée (7, 12 ou 3) présentent en section transversale la forme d'une barbe.

6. Prothèse de valvule cardiaque selon une des revendications précédentes, **caractérisée en ce que** les évidements additionnels (8, 9) sont calculés à une dimension telle que la mise en place du clapet (3) de la valvule puisse s'effectuer sans déformation notable du corps annulaire (1).

7. Prothèse de valvule cardiaque selon une des revendications précédentes, **caractérisée en ce que** la deuxième butée (7) est réalisée sous la forme d'un échelon, qui s'étend à l'intérieur du corps annulaire (1) dans une direction tangentielle le long de la paroi interne, dans une région comprise entre les deux portées de rotation, et qui limite le mouvement du clapet dans le sens allant au-delà de la position fermée.

8. Prothèse de valvule cardiaque selon une des revendications précédentes, **caractérisée en ce qu'**au moins le corps annulaire (1) est muni d'un revêtement (14) fait d'une matière semiconductrice, la matière semiconductrice étant amorphe, notamment microcristalline.

9. Prothèse de valvule cardiaque selon la revendication 8, **caractérisée en ce que** le revêtement (14) présente une épaisseur de l'ordre de 1 $\mu$m, au maximum de 10 $\mu$, avec une rugosité de l'ordre d'environ 0,1 $\mu$.

10. Prothèse de valvule cardiaque selon une des revendications 8 et 9, **caractérisée en ce que** la densité d'état du revêtement (14) est faible au niveau énergétique des états protéiniques, ce qui évite les processus d'échange direct de charges entre les protéines spécifiques de la coagulation et la surface (15) de l'implant.

11. Prothèse de valvule cardiaque selon la revendication 8, **caractérisée en ce que**

le revêtement (13) est composé de carbure de silicium pyrolytique ou amorphe (a-Sic:H), le carbure de silicium amorphe étant notamment formé du produit de dissociation d'un mélange contenant du (SiH4) et/ou du méthane (CH4), ou

en ce que le revêtement (13) est composé de nitrure de silicium amorphe (A-SiN:H) ou d'oxyde de silicium (a-SiO:H).

Fig. 1

Fig.2

Fig. 3a
geschlossen

Fig. 3b
offen

Fig. 4